# EUROPÄISCHE PATENTSCHRIFT

(11) **EP 2 606 913 B1**
(45) Veröffentlichungstag und Bekanntmachung des Hinweises auf die Patenterteilung: **03.01.2018**
(21) Anmeldenummer: 12192022.7
(22) Anmeldetag: 09.11.2012
(51) Int. Cl.: A61L 2/07

(54) **Autoklaventür mit Anschlüssen**
Autoclave door with ports
Autoclave porte avec connecteur

(30) Priorität: 22.11.2011 DE 102011086848
(43) Veröffentlichungstag der Anmeldung: 26.06.2013
(73) Patentinhaber: MELAG Medizintechnik oHG, 10829 Berlin (DE)
(72) Erfinder: Creutzburg, Kai-Hendrik, 12487 Berlin (DE); Podolski, Denis, 13585 Berlin (DE); Bonatz, Stefan, 12623 Berlin (DE); Hartrodt, Matthias, 16341 Panketal (DE)
(74) Vertreter: Maikowski & Ninnemann Patentanwälte Partnerschaft mbB

(56) Entgegenhaltungen:
- EP-A1- 0 294 516
- EP-A1- 0 604 387
- EP-A1- 2 500 041
- WO-A1-00/59552
- WO-A1-2011/101213
- FR-A1- 2 725 135

## Beschreibung

Die vorliegende Erfindung betrifft ein bewegliches Teil mit Mitteln zum Anbringen eines Behälters für medizinische und zahnmedizinische Instrumente in einem Autoklaven gemäß dem Oberbegriff des Anspruchs 1, einen Autoklaven gemäß Anspruch 14 und ein Verfahren zur hygienischen Aufbereitung medizinischer und zahnmedizinischer Instrumente gemäß Anspruch 15.

Es ist bekannt, zum Zwecke der hygienischen Aufbereitung von medizinischen und zahnmedizinischen Instrumenten, die Schritte der Reinigung und/oder der Desinfektion bzw. Sterilisation der besagten Instrumente in Autoklaven, insbesondere in kleindimensionierten Autoklaven, die zur Verwendung in Arztpraxen geeignet sind, durchzuführen. Derartige Autoklaven arbeiten üblicherweise mit Wasserdampf und unter hohem Druck um eine gewünschte Sterilisation bzw. Desinfektion zu erreichen. Es ist ebenfalls bekannt, den Schritt der Sterilisation bzw. Desinfektion kombiniert mit der Reinigung der medizinischen und zahnmedizinischen Instrumente unter Verwendung von geeigneten Reinigungs-bzw. Desinfektionsmitteln durchzuführen.

Um insbesondere zahnmedizinische Instrumente, wie zum Beispiel Bohrer, Haken, Absaugstutzen und ähnliches in geeigneter Weise zu reinigen und zu desinfizieren, ist es bekannt, diese zahnmedizinischen Instrumente in separaten in den Autoklaven einbringbare Kassetten bzw. Behälter zu lagern. Die hygienische Aufbereitung von zahnmedizinischen Übertragungsinstrumenten stellt dabei eine besondere Herausforderung dar. So ist es insbesondere für die Reinigung solcher Instrumente unerlässlich, die engen Lumen der Instrumente ausreichend durchzuspülen. Um dies zu erreichen werden die Übertragungsinstrumente in speziellen Kassetten angeordnet. Solche Kassetten bzw. Behälter zur Aufbereitung von medizinischen Instrumenten, insbesondere von zahnärztlichen Übertragungsinstrumenten, sind zum Beispiel aus EP 870 512 B1 und EP 638 298 B1 bekannt.

In der EP 870 512 B1 wird die Verwendung von Kassetten beschrieben, in denen Adapter angeordnet sind, in welche die in der Regel Hohlräume aufweisenden medizinischen oder zahnmedizinischen Instrumente eingeführt werden können, wobei anschließend durch eine geeignete Flüssigkeitszufuhr durch die Adapter und im Folgenden durch die Hohlräume der eingeführten Instrumente letztere mit Reinigungsflüssigkeit in adäquater Weise gereinigt werden können.

Aus der WO 2011/101213 A1 ist ein Behälter für zahnärztliche Instrumente zur Verwendung in einem Wiederaufbereitungsgerät entnehmbar, der über in den Deckel des Gerätes integrierte Verbindungsrohre mit einem Versorgungssystem fluidisch gekoppelt ist; das Zuführen von Fluiden erfolgt demnach über die Tür des Aufbereitungsgeräts. Dabei erfolgt das Einsetzen der Kassette bzw. des Behälters entlang der Innenwand des Wiederaufbereitungsgerätes geführt; Kassette und Innenraum des Wiederaufbereitungsgerätes weisen demnach fast gleiche Volumina auf. Die Kassette hat die auch Eigenschaft, dass das Öffnen und Schließen automatisch im Aufbereitungsgerät erfolgt, um die Verletzungsgefahr zu minimieren.

Das in der US 2006/0196728 A1 beschriebene Aufbereitungsgerät für Übertragungsinstrumente verfügt über Anschlüsse in der Tür des Gerätes, welche ein Zu- und Abführen der Prozessmedien über die Tür ermöglichen.

Nachteilig bei den in den genannten Druckschriften beschriebenen Kassetten ist, dass die beschriebenen Kassetten lediglich in speziell für diese Kassetten ausgelegten Geräten, das heißt für die Kassetten spezifisch konstruierte Autoklaven, verwendet werden können; diese Autoklaven bieten außerhalb der Kassetten keinen weiteren Nutzraum für andere zu sterilisierende Geräte oder Instrumente. Dadurch wird die Nutzbarkeit und Einsetzbarkeit der Autoklaven zur Aufbereitung von Standardinstrumenten, vor allem im größeren Umfange stark eingeschränkt.

Zur Vermeidung dieses Problems wird in der WO 2006/126103 A2 ein weitergehender Ansatz beschrieben. So wird hier ein Autoklav vorgestellt, der mittels eines, in den Autoklaven einführbaren, zusätzlichen Behälters zur Aufbereitung von zum Beispiel zahnmedizinischen Instrumenten umgerüstet werden kann. Die Dimensionen des Behälters sind derart ausgelegt, so dass dieser Behälter im Autoklaveninnenraum an der Rückseite des Autoklaven, das heißt, an der der Autoklaventür gegenüberliegenden Seite, befestigt werden kann. Der Behälter ist dabei druckfest ausgelegt, wodurch eine hermetische Abriegelung zwischen der Autoklavenkammer und dem zusätzlichen Behälter erfolgen kann. Diese Ausgestaltung bietet den Vorteil, dass durch etwaige Verschmutzungen an den medizinischen und zahnmedizinischen Instrumenten, die im Verlauf der Reinigung und Sterilisation entfernt werden, sowie durch eventuelle Pflegemittelreste keine Kontamination des gesamten Autoklavenraums erfolgt, so dass der Autoklav uneingeschränkt zur Aufbereitung von anderen Standardinstrumenten einsetzbar ist.

Wie erwähnt, erfolgt die Befestigung des Behälters bzw. der Kassette gemäß der WO 2006/126103 A2 am Boden oder der hinteren Wand der Autoklavenkammer, wodurch auch die Adaptierung bzw. Zufuhr der Prozessmedien wie Reinigungsmittel und Wasser, am Boden oder der hinteren Wand der Autoklavenkammer erfolgt. Bei Verwendung einer derartigen Aufbereitungskassette in einem Standardautoklaven ist diese Art der Befestigung jedoch von Nachteil, da die typischerweise relativ kleinvolumige Kassette sehr tief in den Autoklaveninnenraum eingeführt werden muss. Insbesondere im Falle von Autoklaven mit Mantelheizung oder Doppelmanteltechnologie ist eine solche Befestigung somit mit hohen Sicherheitsrisiken verbunden. So besteht bei der Handhabung des Behälters bzw. der Kassette ein hohes Risiko von Verbrennungen auf Seiten des Bedienpersonals.

EP 604 387 A1 beschreibt einen Autoklaven mit einer Temperatur-Messanordnung, in welchen Beschickungswagen mit dem Sterilisationsgut in axialer Richtung eingefahren werden. Die spezifische Messanordnung mit einem Temperaturmessfühler und einer Messelektronik ist in einem thermisch isolierten Behälter angeordnet. Der Behälter verfügt über eine außerhalb des Behälters an deren Deckel angeordnete Sendeantenne.

EP 294 516 A1 offenbart eine Vorrichtung zur Reinigung von Hygienegefäßen mit einer Klapptür, wobei die Klapptür in ihrem inneren Hohlraum ein Rohrleitungsverbindungssystem mit zwei oder mehreren auf der Innenseite der Tür angeordneten Düsen aufweist, durch welche Wasser in des Innere der Vorrichtung eingesprüht werden kann. Auch weist die Klapptür ein gebogenes Rohrstück auf, auf welches eine zu reinigende Flasche aufgeschoben wird.

FR 2 725 135 zeigt eine Vorrichtung zur Desinfektion (z.B. Autoklav) mit einer Tür auf, wobei jedoch kein separater Instrumentenbehälter verwendet wird.

EP 2 500 041 A1 offenbart ein Gerät zum Reinigen und Pflegen von ärztlichen Instrumenten mit einer Reinigungskammer in der sich Halterungen zur Aufnahme der Instrumente befinden. Die Instrumente ragen unmittelbar von den Halterungen in die Reinigungskammer hinein.

WO 00/59552 A1 betrifft eine Kassette für eine Sterilisationsvorrichtung. Die Kassette verfügt über eine Einlasskupplung und eine Auslasskupplung, die an der Rückseite der Kassette vorgesehen sind.

Ebenfalls ist es problematisch, dass sich die Instrumente in dem Behälter nach Befestigung im Autoklaven sehr stark erhitzen, wenn nicht unverzüglich eine Aufbereitung erfolgt. Unter diesen Bedingungen können die Instrumente beschädigt werden und Verschmutzungen können an den sich in dem Behälter bzw. der Kassette befindlichen Instrumenten fixiert werden, wodurch eine sinnvolle Reinigung und Sterilisation der Instrumente erschwert wird bzw. unmöglich gemacht wird.

Darüber hinaus ist eine Kupplung bzw. Befestigung eines Instrumentenbehälters am Boden des Autoklaven mit einem sehr hohen fertigungstechnischen Aufwand verbunden. Zusätzlich ist eine optimierte Kühlung für eine schnelle Wiederverfügbarkeit der Instrumente unerlässlich, wobei die in den zitierten Dokumenten beschriebenen Systeme hierfür nur einen sehr begrenzten Spielraum zulassen.

Auch sollte es den Benutzer in Form des geschulten Bedienpersonals ermöglicht werden, sich vom korrekten Sitz der Kassette im Autoklaven überzeugen zu können. Dies ist insbesondere bei Verwendung von druckfesten, hermetisch abgeriegelten Kassetten von Bedeutung, in denen ein kompletter Aufbereitungszyklus stattfinden soll, da die erfolgreiche Reinigung und Sterilisation nur bei einwandfreier Zuführung der Flüssigkeitsmedien gewährleistet werden kann.

Auch dies ist im Falle der Befestigung einer Instrumentenkassette am Boden oder der hinteren Wand eines Autoklaven nur mit hohem technischem Aufwand möglich.

Der vorliegenden Erfindung liegt daher die Aufgabe zugrunde, die aus dem Stand der Technik bekannten Nachteile zu überwinden und eine Anordnung für einen Autoklaven bereitzustellen, die eine sichere und bedienerfreundliche Kupplung einer Instrumentenkassette bzw. eines Instrumentenbehälters mit dem Autoklaven ermöglicht und zudem eine Optimierung der Abkühlzeit und des Fertigungsaufwandes ermöglicht.

Erfindungsgemäß wird diese Aufgabe mit einem Autoklaven nach Anspruch 1 gelöst.

Entsprechend wird ein Autoklav mit einem Autoklavenkessel und einer Autoklaventür bereitgestellt, wobei in oder an der Autoklaventür des Autoklaven Mittel zum Halten von mindestens einem Behälter für ärztliche und zahnärztliche Instrumente innerhalb des Autoklaven und Anschlüsse zum Einführen und Abführen von Fluiden in und aus dem Behälter angeordnet sind. Der mindestens eine Behälter für ärztliche und zahnärztliche Instrumente ist als verschließbare Kammer ausgebildet und lösbar innerhalb des Autoklavenkessels angebarcht.

Entsprechend ist die an der Autoklaventür I vorgesehene Anordnung aus Haltemitteln und Anschlüssen zum Einführen und Abführen von Fluiden zum lösbaren Anbringen von mindestens einem Behälter für medizinische und zahnmedizinische Instrumente innerhalb eines Autoklaven geeignet.

Die Autoklaventür liegt in schwenkbarer Form vor und ermöglicht dasden Zugriff durch Personen in den Innenraum des Autoklaven.

Der vorliegende Autoklav weist verschiedene Vorteile gegenüber den aus dem Stand der Technik bekannten Vorgehensweisen auf. So ermöglicht die vorliegende Anordnung ein ergonomisches Anbringen und Ablösen des Behälters für medizinische und zahnmedizinische Instrumente. Da der Behälter mit den medizinischen und zahnmedizinischen Instrumenten an einem beweglichen Teil, insbesondere der Tür des Autoklaven, angebracht ist, ist das Anbringen und Ablösen des Behälters mit einem minimierten Risiko von Verbrennungen möglich.

Die Abkühlung des Instrumentenbehälters kann in unkomplizierter Weise durch Öffnen der Autoklaventür erfolgen. Somit ist eine beschleunigte Kühlung zum Beispiel durch eine automatische Türöffnung möglich.

Aufgrund der Anordnung der Anschlüsse zum Einführen und Abführen von Fluiden in oder an der Autoklaventür, ist ein wartungsfreundlicher Zugang zu diesen wichtigen Bauteilen möglich, wobei eine einfache optische Kontrolle des Zustandes der Anschlüsse bzw. Anschlussstücke und der Verschlussmechanismen
ausreichend ist. Zudem erfordern die verwendeten Anschlussstücke bzw. Kupplungselemente keine aufwendige Fertigung, sondern
einfache, aus dem Stand der Technik bekannte Anschlusstücke sind zum Einsatz in der erfindungsgemäßen Anordnung ausreichend.

Ein weiterer Vorteil der vorliegenden Anordnung besteht darin, dass nach dem Befestigen des Instrumentenbehälters am Autoklaven, genauer an der Autoklaventür, kritische Temperaturen im Instrumentenbehälter vermieden werden, so dass der sich anschließende Reinigungs- und Sterilisationsvorgang in optimaler Weise durchgeführt werden kann. Auch ermöglicht die vorliegende Anordnung eine einfache Kontrolle des korrekten Anbringens des Instrumentenbehälters.

Bevorzugterweise weist der zum Einsatz kommende Autoklavenkessel ein im Vergleich zu dem mindestens einen Behälter für ärztliche und zahnärztliche Instrumente größeres Volumen auf. Demnach ist ein kleinerer Behälter zur Aufnahme von ärztlichen und zahnärztlichen Instrumenten innerhalb eines größeren Volumeninnenraums Autoklaven vorgesehen, so dass der Behälter nicht mit den Innenwänden des Autoklaveninnenraums in Kontakt tritt, sondern vielmehr ein genügend großer Abstand zwischen dem Behälter und den Innenwänden des Autoklaven existieren. Dies ermöglicht eine ergonomische und sichere Handhabung des Behälters.

So kann das Volumen (Kammervolumen) des Autoklavenkessels bei 12 L oder darüber liegen, was den Dimensionen eines Tischautoklavens entspricht.

Der Behälter zur Aufnahme der ärztlichen und zahnärztlichen Instrumente weist hingegen ein deutlich kleineres Volumen als das Kammervolumen des Autoklavenkessels auf. So können Behälter mit einem Volumen von weniger als 7 L, bevorzugt weniger als 5 L, insbesondere bevorzugt weniger als 4 L verwendet werden.

Entsprechend beträgt in einer bevorzugten Ausführungsform das Verhältnis des Volumens des mindestens einen Behälters für ärztliche und zahnärztliche Instrumente und Volumen des Autoklavenkessels mindestens 1 : 1,7, bevorzugt 1:2,5, insbesondere bevorzugt 1:3,4.

Auch ist es bevorzugt, wenn die Abmaße des Behälters zur Aufnahme der ärztlichen und zahnärztlichen Instrumente, wie z.B. Höhe, Breite und/oder Durchmesser, geringer sind als die vergleichbaren Abmaße des beweglichen Teils des Autoklaven, an dem der Behälter befestigt werden kann. Nur in solch einem Fall ist eine Anbringung des Behälters an einem beweglichen Teil des Autoklaven wie z.B. der Autoklaventür praktikabel und durchführbar.

Demnach ist es vorstellbar, dass der Aufnahmebehälter so dimensioniert ist, dass dieser bis zu 75% der Fläche des beweglichen Teils bedeckt bzw. dass der Aufnahmebehälter mit einer seiner Behälterseiten an bis zu 75% der Fläche des beweglichen Teils anliegt. Bevorzugterweise kann diese bedeckte Fläche des beweglichen Teils bis zu 60%, insbesondere bevorzugt bis zu 40% betragen. Mit anderen Worten, es ist insbesondere bevorzugt, wenn der Behälter etwa 40% der Fläche des beweglichen Teils wie einer Autoklaventür in Anspruch nimmt. Es ist offensichtlich, dass die Größe der von dem Aufnahmebehälter abgedeckten Fläche des beweglichen Teils durch die Form und die Dimensionen des Aufnahmebehälters bestimmt wird. So kann der Aufnahmebehälter quaderförmig aber auch halbrund oder ähnlich ausgebildet sein.

In einer Ausführungsform des vorliegenden beweglichen Teils sind die Haltemittel und die Anschlüsse zum Ein- und Abführen von Fluiden in und aus dem Behälter, wie dem Instrumentenbehälter, gemeinsam auf einer Trägerplatte oder auf separaten Trägerplatten angeordnet, die jeweils an dem beweglichen Teil, das heißt bevorzugt an der Autoklaventür, anbringbar sind. Mit anderen Worten, Haltemittel und Anschlüsse können gemäß dieser Ausführungsform auf einer von der Autoklaventür entfernbaren Platte bzw. entfernbarem Trägermaterial angeordnet sein. Die Trägerplatte bzw. das Trägermaterial wäre dann in geeigneter Weise an dem beweglichen Teil des Autoklaven, wie zum Beispiel an der Autoklaventür, befestigt, wobei in entsprechender Weise Öffnungen für das Ein- und Abführen von Fluiden in der Trägerplatte und in dem beweglichen Teil vorgesehen sind. Eine derartige Anordnung weist den Vorteil auf, dass Haltemittel und Anschlussstücke bzw. Kopplungsstücke bei Abnutzung einfach entfernbar sind und ebenfalls leicht zu reinigen sind. Auch ist die Verwendung einer separaten Trägerplatte aus fertigungstechnischer Sicht von Vorteil; z.B. ist eine Baugruppenfertigung mit höherer Genauigkeit in den Anschlussdimensionen möglich.

In einer weiteren Ausführungsform ist es jedoch möglich, dass die Haltemittel und die Anschlussstücke für die Anschlüsse zum Ein- und Abführen von Fluiden in und aus dem Behälter einstückig mit dem beweglichen Teil, insbesondere mit der Autoklaventür ausgebildet sind. Hierbei sind die Haltemittel und Anschlussstücke bevorzugt einstückig in der Türronde der Autoklaventür ausgebildet und können in Form eines Schweißteils oder eines Druckgussbauteils vorliegen. Auch ist es denkbar, die einstückige Ausbildung des vorliegenden beweglichen Teils ist mittels einer Kombination aus Schweißteil und Druckgussteil herzustellen.

Bevorzugterweise ist das zum Einsatz kommende, mindestens eine Haltemittel ein Mittel, das eine Rotationsbewegung des Behälters ermöglicht, um so den Behälter mittels einer geeigneten Rotationsbewegung in die entsprechende Endposition beim Anbringen des Behälters geführt wird. Das Haltemittel ist bevorzugterweise in Form eines Bolzens oder einer Schraube ausgebildet, es ist jedoch auch möglich, dass das Haltemittel z.B. in Form einer Aussparung derart ausgebildet ist, so dass ein Bajonetteingriff zwischen Haltemittel und Instrumentenbehälter ermöglicht wird. Ein Bajonettverschluss ist dabei eine schnell herstell- und lösbare mechanische Verbindung zweier zylindrischer Teile in ihrer Längsachse. Die Teile werden durch Ineinanderstecken und entgegengesetztes Drehen verbunden und so auch wieder getrennt.

Das Haltemittel dient somit zum einen dem Halten des Gewichtes des Instrumentbehälters und ermöglicht gleichzeitig eine zum Beispiel rotierende Kupplungsbewegung, wodurch der Instrumentenbehälter durch die Haltemittel in die gewünschte Endposition an der Autoklaventür geführt wird. Zusätzliche Führungsgestelle, wie dies etwa bei einer Kupplung am Boden des Autoklaveninnenraums erforderlich ist, müssen daher nicht mehr vorgesehen werden. Es handelt sich somit bei den erfindungsgemäß verwendeten Haltemitteln um eine Kombination von Haltemittel und Führungsmittel.

Grundsätzlich sind jedoch auch weitere Ausführungsformen des Haltemittels denkbar, wie zum Beispiel eine Halteplatte oder eine Kombination verschiedener Halterungs- und Führungsmittel. Bevorzugterweise bestehen die Haltemittel aus Edelstahl oder auch entsprechende Kunststoffe, die den gestellten Materialanforderungen Rechnung tragen.

Um eine ausreichende Halterung des Instrumentenbehälters an der Autoklaventür zu gewährleisten, sind bevorzugterweise am Instrumentenbehälter zu den Haltemitteln der vorliegenden Anordnung korrespondierende Mittel vorgesehen, die eine Halteverbindung zwischen Instrumentenbehälter und beweglichen Teil ermöglichen. So können am Instrumentenbehälter Mittel zum Eingreifen in das Haltemittel der vorliegenden Anordnung oder geeignete Kopplungsstücke vorgesehen sein. Derartige Kopplungsstücke sind dem Fachmann allgemein bekannt.

Gemäß einer bevorzugten Ausführungsform sind die Anschlüsse zum Einführen und Abführen von Fluiden, die in oder an dem mindestens einem beweglichen Teil des Autoklaven, insbesondere an der Autoklaventür, vorgesehen sind, dafür geeignet, um eine dichtende Verbindung bzw. Kupplung mit den am Behälter vorgesehenen Anschlüssen zum Ein- und Abführen von Fluiden herzustellen. Somit sind also die in dem beweglichen Teil angeordneten Anschlussstücke kompatibel mit den Anschlussstücken des Instrumentenbehälters und ermöglichen zudem die Herstellung einer dichten Verbindung zwischen beweglichem Teil, wie z.B. der Autoklaventür, und dem Instrumentenbehälter. Es ist auch möglich, dass die diskutierten Anschlussstücke zusätzliche Mittel umfassen, um eine dichtende Verbindung zwischen Behälter und den Fluidanschlüssen im beweglichen Teil zu gewährleisten. So kann die dichtende Verbindung manuell, pneumatisch oder anderweitig automatisch herstellbar sein. Die Herstellung der dichtenden Verbindung erfolgt dabei bevorzugterweise nachdem die Kassette bzw. der Instrumentenbehälter seine Endposition nach Einhängen des Instrumentenbehälters an der Autoklaventür erreicht hat. Bevorzugterweise bestehen die Anschlussstücke aus Edelstahl oder auch entsprechende Kunststoffe, die den gestellten Materialanforderungen Rechnung tragen.

Bevorzugterweise sind die in oder an dem beweglichen Teil angeordneten Anschlüsse zum Einführen und Abführen von Fluiden in Form von separaten Stutzen ausgebildet oder sind in ein kumuliertes Kupplungsstück integriert. Solch ein kumuliertes Kupplungsstück besteht bevorzugterweise aus einem Stück, bevorzugterweise aus Edelstahl oder geeigneten Kunststoffmaterial, in welches die Fluidanschlussstücke integriert sind, und welches mittels geeigneter Befestigungsmittel wie z.B. Schraube oder Bolzen mit dem beweglichen Teil, bevorzugt unter Verwendung eines zwischen Kupplungsstück und beweglichen Teil angeordneten Verbindungsteil verbunden ist. Die geometrische Form des Kupplungsstücks ist derart, dass dieses in ein am Instrumentenbehälter vorgesehenes Haltemittel z.B. in Form einer Aussparung eingreifen kann. Das zum Kupplungsstück korrespondierende Haltemittel am Instrumentenbehälter ist so gestaltet, dass der Instrumentenbehälter zunächst in einer geraden Bewegung hin zum Kupplungsstück am beweglichen Teil des Autoklaven bewegt wird, wobei das Kupplungsstück in das korrespondierende Haltemittel z.B. in Form einer sich entlang einer vertikalen Linie des Instrumentenbehälters nach oben hin verjüngenden Aussparung eingeschoben wird, und der Instrumentenbehälter anschließend senkrecht zur Einschubrichtung nach oben in Richtung zur Verjüngung in der Aussparung am Instrumentenbehälter bewegt wird, wodurch sich der gewünschte Eingriff zwischen Haltemittel am Instrumentenbehälter und Kupplungsstück am beweglichen Teil ergibt. Das Einschieben oder Aufschieben des Instrumentenbehälters auf das Kupplungsstück kann auch mit einer Rotationsbewegung gekoppelt sein.

Die angeführten Stutzen bzw. Kupplungsstück gewährleisten ein selbsttätiges Ankuppeln der Fluidzuführung, das heißt der Anschlussstücke an der Autoklaventür und Instrumentenbehälter, durch die Gewichtskraft des Behälters. Der Behälter weist entsprechend bevorzugterweise zu den Anschlussstücken in der Autoklaventür korrespondierende Nuten oder Flansche auf.

Der Instrumentenbehälter ist bevorzugt als verschließbare Kammer ausgebildet, die einem prozessbedingten Überdruck von bis zu 0,5 bar standhalten kann. Es ist aber auch denkbar, den Instrumentenbehälter in Form einer insbesondere unter Sterilisationsbedingungen druckfesten Kammer zu gestalten, so dass eine hermetische Abriegelung zwischen Autoklavenkammer und Instrumentenbehälter möglich ist. Ein hermetisch geschlossener Instrumentenbehälter erlaubt eine separate Bedienung desselbigen unabhängig von der Bedienung des restlichen Autoklaven, so dass bei Bedarf lediglich der Instrumentenbehälter benutzbar ist, und nicht der gesamte Autoklav zur Reinigung von z.B. einigen zahnmedizinischen Instrumenten verwendet werden muss. Der Instrumentenbehälter ist bevorzugt aus einem autoklavierbaren Polymerwerkstoff hergestellt, kann jedoch auch aus jedem weiteren geeigneten Material bestehen.

In einer bevorzugten Ausführungsform ist innerhalb des Instrumentenbehälters ein Trägersystem umfassend eine Trägerplatte und daran ausgebildete Adapter angeordnet. Die Adapter dienen der Verbindung der medizinischen oder zahnmedizinischen Instrumente mit dem Trägersystem. Zum Beispiel können die medizinischen oder zahnmedizinischen Instrumente auf die entsprechenden Adapter aufgesteckt werden. In einer besonders bevorzugten Ausführungsform sind die Adapter an der Trägerplatte derart orientiert, so dass Prozessmedien, wie zum Beispiel geeignete Reinigungsflüssigkeiten durch die Instrumente hindurch fließen können. Entsprechende Adapter sind unter anderem in der EP 870 512 B1 beschrieben.

In einer Ausführungsform der vorliegenden Erfindung umfasst das vorliegende bewegliche Teil des Autoklaven bzw. die vorliegende Anordnung mindestens ein Mittel zum Erkennen des korrekten Anbringens des Behälters, wobei das Mittel an dem Instrumentenbehälter, an dem beweglichen Teil und/oder an dem Autoklaven, insbesondere Autoklavenkessel, vorgesehen ist. Ein derartiges Mittel kann zum Beispiel in Form eines Schalters verbunden mit einer geeigneten Steuereinrichtung ausgebildet sein. Eine weitere Möglichkeit der Ausbildung eines solchen Mittels besteht in einem Hebel, der zum Beispiel auf der Oberseite am Instrumentenbehälter selber angebracht ist und ausschließlich in einer vorbestimmten Position das Schließen der Tür erlaubt. Befindet sich dieser Hebel zum Beispiel nicht in dieser vorbestimmten Position, ist eine Schließung des Autoklaven nicht möglich. Bei Erreichen der Endposition des Instrumentenbehälters an dem beweglichen Teil kann dieser Hebel entsprechend in die vorbestimmte Position gebracht werden, wie zum Beispiel in einer Einkerbung bzw. Vertiefung auf der Oberseite des Instrumentenbehälters eingebracht werden, so dass dann ein Verschließen der Autoklaventür mit dem Autoklavenkessel möglich ist.

In einer weiteren Ausführungsform umfasst die vorliegende Anordnung mindestens ein Mittel zum Verriegeln des Instrumentenbehälters in seiner zum Autoklavieren vorgesehenen Endposition, wobei dieses Verriegelungsmittel an dem Instrumentenbehälter selber, an dem beweglichen Teil, wie der Autoklaventür oder an dem Autoklavenkessel, vorgesehen sein kann. Dieses Mittel kann in Form eines manuellen oder automatisch zu betätigenden Mechanismus, wie zum Beispiel einem einfach umzulegenden Hebel oder einem Schnappverschluss, ausgebildet sein.

Es ist auch denkbar, dass das Verriegelungsmittel nicht nur mittels eines mechanischen Mechanismus eine Verbindung zwischen Aufnahmebehälter und beweglichen Teil wie z.B. der Türronde einer Autoklaventür ermöglicht, sondern dass die Verbindung auch mittels eines magnetischen Mechanismus bewirkbar ist. So kann ein magnetisch wirkendes Verriegelungsmittel in einfacher Weise an dem beweglichen Teil befestigt werden. Aufgrund der magnetischen Wirkung des Verriegelungsmittels wird der Aufnahmebehälter, der in diesem Fall vorteilhafterweise zumindest teilweise aus einem magnetisierbaren Material besteht, an dem beweglichen Teil stationär angeordnet, kann jedoch auch in einfacher Weise wieder von dem beweglichen Teil entfernt werden.

Die Verriegelung kann, wie gesagt, manuell aber auch automatisch zum Beispiel beim Schließen der Tür erfolgen. Ein Fachmann wird erkennen, dass sich nahezu jede aus dem Stand der Technik bekannte Verriegelungsvorrichtung für den Zweck der vorliegenden Erfindung verwenden lässt.

Bevorzugterweise sind das Mittel zum Erkennen des korrekten Anbringens des Behälters und das Mittel zum Verriegeln des Behälters in seiner zum Autoklavieren vorgesehenen Endposition kombiniert ausgebildet. Das heißt, das Erkennungsmittel für das korrekte Anbringen des Instrumentenbehälters als auch das Verriegelungsmittel können in einem Stück ausgebildet sein, das heißt, sie können durch ein und dasselbe Mittel, wie zum Beispiel einen Hebel, ausgebildet sein. Dies vereinfacht den Gesamtaufbau der vorliegenden Anordnung.

In einer weiteren Ausführungsform des vorliegenden Autoklaven wird das in den Behälter ein- und ausgeführte Fluid über mindestens eine Leitung aus dem Autoklavenkessel über die hierfür am beweglichen Teil des Autoklaven und dem Instrumentenbehälter vorgesehenen Anschlüsse bzw. Anschlussstücke in den Behälter ein- und ausgeführt. Die ein- und ausgeführten Fluide werden auch als Prozessmedien bezeichnet, wobei unter dem Begriff der Prozessmedien insbesondere Reinigungsflüssigkeiten, Desinfektionsflüssigkeiten, Pflegemittel, Wasser und Wasserdampf zu verstehen sind.

In der soeben beschriebenen Ausführungsform läuft die Leitung bevorzugterweise entlang des Autoklavenkessels innerhalb der Gehäuseummantelung. Das heißt, die Leitung bzw. Leitungen, die insbesondere in Form von Schläuchen ausgebildet, werden entlang der Wand des Autoklaven vom hinteren Abschnitt desselbigen hin zum vorderen Abschnitt des Autoklaven am Türscharnier zur Autoklaventür als beweglichen Teil
geführt. Da die Autoklaventür ein bewegliches Teil der Autoklavenkammer darstellt, muss an diesem Punkt sichergestellt werden, dass die fluidführenden Leitungen, insbesondere fluidführende Schläuche, keinen starken Dehnungen oder Stauchungen aufgrund der Bewegung der Autoklaventür während des Öffnens und Schließens unterliegen.

Zu diesem Zweck ist es in einer Ausführungsform vorgesehen, dass die mindestens eine Leitung an mindestens zwei Halterungen unter Verwendung von geeigneten Befestigungsmitteln am Autoklaven befestigt ist. In dieser Ausführungsform ist mindestens eine erste Halterung am beweglichen Teil des Autoklaven, wie der der Autoklaventür, und mindestens eine zweite Halterung am Autoklavengehäuse, das heißt auf der Außenseite des Autoklavenkessels, angeordnet. Durch die Fixierung der Fluidleitung an mindestens zwei Punkten wird eine einseitige Zugentlastung umgesetzt. Aufgrund der beschriebenen Anordnung der Fluidleitung unter Verwendung von mindestens zwei Halterungen kommt der durch die mindestens eine Leitung gebildete Halbkreis in der Fluchtlinie eines Scharniers des beweglichen Teiles, wie einem Türscharnier, zum liegen. Der erwähnte Halbkreis ergibt sich aus der spezifischen Anordnung bzw. Führung der Fluidleitung vom hinteren Abschnitt des Autoklaven entlang des länglichen Autoklavenkessels hin zur Vorderseite des Autoklaven, das heißt hin zur Tür, die beweglich und im geschlossenen Zustand senkrecht zu der Längsseite des länglichen Autoklavenkessels angeordnet ist. Mit anderen Worten, wenn die Fluidleitung, die kommend von der Hinterseite des Autoklaven die Vorderseite erreicht, ist es, um die erfindungsgemäße Anordnung in der Autoklaventür zu bewirken, notwendig, dass die entlang der Längsseite des Autoklaven geführte Fluidleitung derart an der Vorderseite umgebogen wird, dass die Fluidleitung nunmehr senkrecht zu der Längsseite des Autoklaven entlang der geschlossenen Autoklaventür verläuft. Aufgrund der spezifischen Anordnung der umgebogenen Fluidleitung in den beschriebenen zwei Halterungen ergibt sich der beschriebene Halbkreis. Die Befestigung der Fluidleitung in den zwei Halterungen führt entsprechend zu einer vernachlässigbaren Stauchung oder Streckung der Fluidleitung bei den Öffnungs- und Schließungsvorgängen der Autoklaventür.

Wie bereits oben angedeutet, liegen die Fluidleitungen insbesondere in Form von Schläuchen aus geeignetem Material, wie zum Beispiel hierfür typischerweise verwendeten elastischen Polymermaterialien, vor. Die Schläuche sind bevorzugterweise an den mindestens zwei Halterungen mittels geeigneter Befestigungsmittel, wie zum Beispiel Klemmen, Ringe, Muffen etc. befestigt. Bevorzugterweise umfassen die verwendeten Schläuche eine Ummantelung bzw. Umhüllung, zum Beispiel in Form von Führungsketten oder einen Schutzschlauch, zum mechanischen Schutz und einer optimierten Führung der Schläuche entlang der Längsseite des Autoklavenkessels.

In einer weiteren bevorzugten Ausführungsform der vorliegenden Anordnung ist die mindestens eine Leitung im Bereich zwischen Autoklavenkessel und der Autoklaventür , das heißt zum Beispiel im Bereich des Türscharniers, getrennt, wobei die jeweiligen Endabschnitte der Leitung über geeignete Anschlussstücke zum Verbinden der Endabschnitte der Leitung verfügen. Somit ist es gemäß dieser Ausführungsform nicht notwendig, durchgehende Schläuche zur Führung der Fluide, wie der Prozessmedien, zwischen der Autoklaventür und dem Autoklavenkessel bereitzustellen, sondern die Fluidleitung kann entsprechend im Bereich zwischen dem Autoklavenkessel und der Autoklaventür getrennt sein. Die an den Endabschnitten der Fluidleitung bzw. Schläuche vorgesehenen Anschlussstücke ermöglichen eine Kupplung bzw. Verbindung der getrennten Schläuche bei Bedarf. Dabei ist die Länge der Schläuche entsprechend an die Dimensionen des Autoklaven angepasst, so dass eine Kupplung bzw. Verbindung der Endabschnitte der Fluidleitung möglich ist. Eine derartige Anordnung der Fluidleitung ermöglicht eine wahlweise Anwendung des Instrumentenbehälters durch einfaches Verbinden der Anschlussstücke der Endabschnitte der Leitung.

In einer weiteren Ausführungsform der vorliegenden Anordnung sind an der Außenseite der Autoklaventür, Behältnisse für die in den Instrumentenbehälter ein- und ausgeführte Fluide vorgesehen, so dass die Fluide wie Medienflüssigkeit unmittelbar ausgehend von diesen Behältnissen über die Anschlüsse in der Autoklaventür und im Instrumentenbehälter in den Instrumentenbehälter ein- und ausgeführt werden kann. Das mindestens eine Behältnis für die ein- und ausgeführten Fluide, wie den Prozessmedien, sind somit bevorzugterweise an der äußeren, dem Autoklaveninnenraum gegenüber liegenden Seite der Autoklaventür , zum Beispiel in der äußeren Türverkleidung der Autoklaventür, angebracht. Die Prozessmedien werden also direkt am beweglichen Teil bereitgestellt. Dies hat den Vorteil, dass auf eine Fluidleitung, die die Fluide wie die Prozessmedien aus dem Autoklaveninnenraum entlang der Längsseite des
Autoklavenkessels hin zur Vorderseite zum beweglichen Teil des Autoklaven führen, verzichtet werden kann. Bevorzugt werden Reinigungs- und Pflegeflüssigkeiten oder auch chemische Desinfektionsmittel in den Behältnissen in der Türverkleidung gelagert. Auch ist es denkbar neben den genannten Prozessmedien weitere Mittel zur Gewährleistung der Reinigung wie z.B. eine Umwälzpumpe und/oder Mittel zur Dosierung und Verteilung der Prozessmedien wie z.B. eine geeignete Ventiltechnik in der äußeren Verkleidung des beweglichen Teils unterzubringen.

Der Verschluss des beweglichen Teiles mit dem Autoklavenkessel, wie zum Beispiel der Türverschluss einer Autoklaventür, kann auf verschiedene Weise erfolgen. So kann bei Gestaltung des Verschlusssystems in Form eines Spindelverschlusses eine Möglichkeit zur automatischen Türöffnung und damit eine schnellere Abkühlung der Instrumente umgesetzt werden. Eine weitere Ausführungsform eines Verschlusssystems des beweglichen Teiles verzichtet hingegen auf die Möglichkeit einer automatischen Türöffnung und setzt einen manuell zu betätigenden Türverschluss ein. Ebenfalls ist es denkbar, ein zusätzliches Kühlgebläse innerhalb des Autoklaven vorzusehen, um den Abkühlungsvorgang zu beschleunigen.

Gegenstand der vorliegenden Erfindung ist auch ein Verfahren zur hygienischen Aufbereitung medizinischer und zahnmedizinischer Instrumente, das die folgenden Schritte aufweist:
a) Einbringen von mindestens einem hygienisch aufzubereitenden, medizinischen oder zahnmedizinischen Instrument in einem Behälter für medizinische und zahnmedizinische Instrumente,
b) Koppeln bzw. Verbinden des Behälters mit mindestens einem der oben beschriebenen beweglichen Teile des Autoklaven; das heißt, der Behälter wird in oder an mindestens einem beweglichen Teil des Autoklaven, wie zum Beispiel einer Autoklaventür, unter Verwendung der daran vorgesehenen Haltemittel für den Behälter und Anschlüsse zum Einführen und Abführen von Fluiden in und aus dem Behälter, angeordnet;
c) Durchführen der hygienischen Aufbereitung des sich in dem Behälter befindlichen medizinischen Instruments; eine derartige Aufbereitung kann zunächst die Schritte der Reinigung und Pflege der im Instrumentenbehälter befindlichen Instrumente umfassen mit ggf. anschließender Sterilisation durch Zufuhr von Wasserdampf bei erhöhten Temperaturen bis zu 134 °C und erhöhtem Druck
d) Nach Abkühlen, Ablösen des Behälters von dem beweglichen Teil des Autoklaven; dass heißt der Behälter wird aus der in dem mindestens einen beweglichen Teil des Autoklaven vorgesehenen Anordnung zum Halten des Behälters und der Fluidzufuhr abgenommen; und
e) Entnahme des medizinischen oder zahnmedizinischen Instruments aus dem Behälter.

Bevorzugterweise erfolgt das Abkühlen unter Verwendung eines Kühlmittelstroms, der in dem beschriebenen Instrumentenbehälter vorgesehen ist.

Die Erfindung wird nachfolgend anhand der in der in den Figuren dargestellten beispielhaften Ausführungsformen näher erläutert. Es zeigen:
Fig. 1 ein Autoklavenkessel mit einer ersten erfindungsgemäßen Ausführungsform eines beweglichen Teils mit einem daran befestigten Instrumentenbehälters;
Fig. 2 eine Draufsicht auf die Abbildung der Figur 1;
Fig. 3 eine Ansicht einer weiteren Ausführungsform des erfindungsgemäßen beweglichen Teils;
Fig. 4 eine Ansicht eines Verriegelungsmittels einsetzbar in dem erfindungsgemäßen beweglichen Teil;
Fig. 5 eine Ansicht einer Anordnung von Fluidleitungen zum Ein- und Abführen von Fluiden durch eine Autoklaventür in dem an der Innenseite der Autoklaventür befestigten Instrumentenbehälter; und
Fig. 6 eine Draufsicht auf die Anordnung der Fluidleitung in Figur 5,
Fig. 7a eine Seitenansicht einer weiteren Ausführungsform eines am beweglichen Teil angeordnetes Haltemittel mit korrespondierenden Haltemittel am Instrumentenbehälter vor beiderseitigen Eingriff derselbigen,
Fig. 7b eine weitere Seitenansicht der Ausführungsform der Fig. 7a; und
Fig 7c eine weitere Seitenansicht der Ausführungsform der Fig. 7a nach erfolgtem beiderseitigem Eingriff der Haltemittel,
Fig. 8a eine Seitenansicht einer Ausführungsform eines weiteren Haltemittels, und
Fig 8b eine Seitenansicht einer Ausführungsform eines noch weiteren Haltemittels.

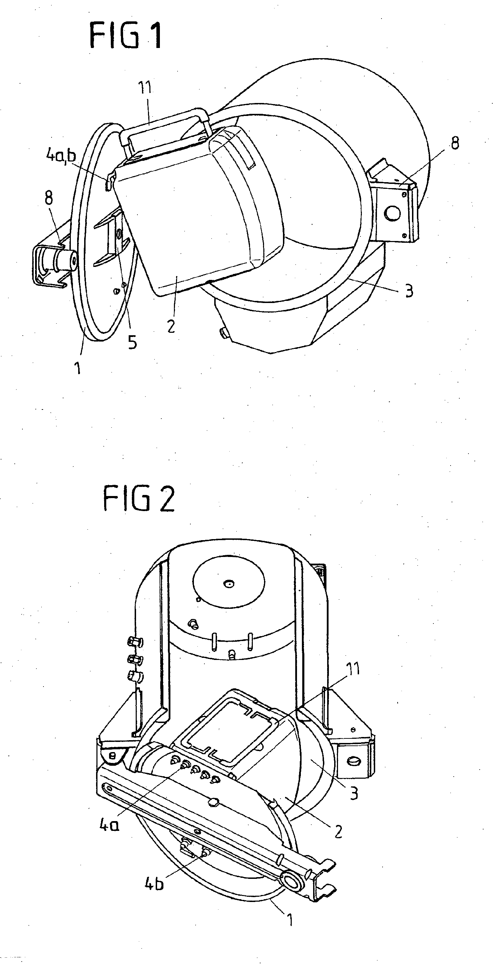

Figur 1 zeigt einen Autoklaven als Vorrichtung zur hygienischen Aufbereitung medizinischer und zahnmedizinischer Instrumente. Der Autoklav weist einen Autoklavenkessel 3 mit einer an dem Kessel mittels eines geeigneten Scharniers befestigten Autoklaventür 1 als bewegliches Teil auf. An der Innenseite der Autoklaventür 1, das heißt die Seite der Autoklaventür, die dem Innenraum des Autoklavenkessels 3 zugewandt ist, ist eine Anordnung zum Befestigen eines Behälters 2 für medizinische und zahnmedizinische Instrumente vorgesehen.

Der Instrumentenbehälter 2 weist an seiner Oberseite einen Hebel 11 auf, welcher ein korrektes Anbringen des Instrumentenbehälters an der Innenseite der Autoklaventür 1 als beweglichem Teil ermöglicht, und somit ein Mittel zum Erkennen des korrekten Anbringens des Behälters an der Autoklaven-Innentür darstellt.

In der in Figur 1 gezeigten Anordnung ist der Vorgang des Anbringens des Instrumentenbehälters 2 an der Innenseite der Autoklaventür 1 in der dafür vorgesehenen Anordnung bestehend aus Haltemittel 5 und Fluidanschluß 4a aufgezeigt, wobei der Hebel 11 als Mittel zum korrekten Anbringen des Instrumentenbehälters 2 hier in einer aufgerichteten Position dargestellt ist, die das korrekte Anbringen und Führen des Instrumentenbehälters 2 an der Autoklaven-Innentür ermöglicht. Wie schematisch der Figur 1 zu entnehmen, weist die Anordnung zum lösbaren Anbringen des Instrumentenbehälters 2 ein Haltemittel 5 in Form eines Kupplungsstückes und Anschlüsse 4a,b zum Einführen und Abführen von Fluiden in und aus dem Instrumentenbehälter 2 auf. In dem vorliegenden Beispiel der Figur 1 ist das Haltemittel 3 mittig an der Innenseite der Autoklaventür 1 angebracht.

Auf der Außenseite der Autoklaventür 1 ist ein länglich geformter horizontal verlaufender Handgriff 13 zum Betätigen der Autoklaventür 1 erkennbar. Dieser Handgriff 13 erfüllt mehrere Zwecke. Zum einen erfolgt über den Handgriff 13 eine Befestigung der Autoklaventür 1 an dem Autoklavenkessel 3 mittels geeigneter Scharniere (siehe hierzu auch Figur 2). Des Weiteren ist in den Handgriff 13 an seinem Ende, das dem Türscharnier gegenüber liegt, ein Türverschluss 8 angebracht, der ein abdichtendes Verschließen der Autoklaventür 1 mit dem Autoklavenkessel 3 ermöglicht. Ein hierfür geeigneter Türverschluss 8 ist bevorzugt in Form eines Spindelverschlusses ausgebildet, der zur automatischen Türöffnung angesteuert werden kann. Für den Fachmann ist dabei jedoch offensichtlich, dass auch weitergehende Formen von Türverschlüssen, wie zum Beispiel manuelle Türverschlüsse, hier durchaus zum Einsatz kommen können.

In Figur 2 ist eine Draufsicht auf die Darstellung der Figur 1 umfassend einen Autoklavenkessel 3 und Autoklaventür 1 mit daran befestigtem Instrumentenbehälter 2 aufgezeigt. Im Unterschied zu der Ausführungsform der Figur 1 ist im Falle der Figur 2 der Instrumentenbehälter 2 bereits vollständig an der Innenseite der Autoklaventür 1 angebracht und verriegelt. Die Anschlussstücke 4a,b zum Ein- und Abführen von Fluiden in und aus dem Instrumentenbehälter 2 können in beliebiger Anzahl, zum Beispiel zwei bis zehn, bevorzugterweise zwei bis sieben, vorgesehen sein.

Über diese Anschlussstücke 4a,b ist es nunmehr möglich, die für die Reinigung und Sterilisation von in dem Instrumentenbehälter 2 angeordneten Instrumenten unter Verwendung von geeigneten Reinigungs- und Pflegemitteln sowie den für die Sterilisation notwendigen Wasserdampf in den Instrumentenbehälter 2 einzuführen. Auch ist es denkbar, dass die dargestellten Anschlüsse 4a,b separat voneinander verwendet werden können, das heißt, dass nicht alle Anschlussstücke 4a,b gleichzeitig verwendet werden, sondern dass einzelne Anschlussstücke bei bestimmten Anwendungen unbenutzt bleiben.

Der in Figur 1 in einer aufrechten Position gezeigte Hebel 11, der zur Führung und dem korrekten Anbringen des Instrumentenbehälters 2 an der Autoklaventür 1 dient, wird nach dem Anbringen des Instrumentenbehälters 2, das heißt nach Erreichen der Endposition des Instrumentenbehälters 2 an der Autoklaventür 1, entsprechend zur Seite geschoben bzw. in die auf der Oberseite des Instrumentenbehälters 2 hierfür vorgesehenen Vertiefungen hineingedrückt. Dies ist notwendig, da der Hebel 11 ansonsten ein Schließen der Autoklaventür 1 verhindern würde. Wie bereits mehrfach erläutert stellt der Hebel 11 nur eine mögliche Ausführungsform eines geeigneten Mittels zum Führen und Verriegeln des Instrumentenbehälters dar.

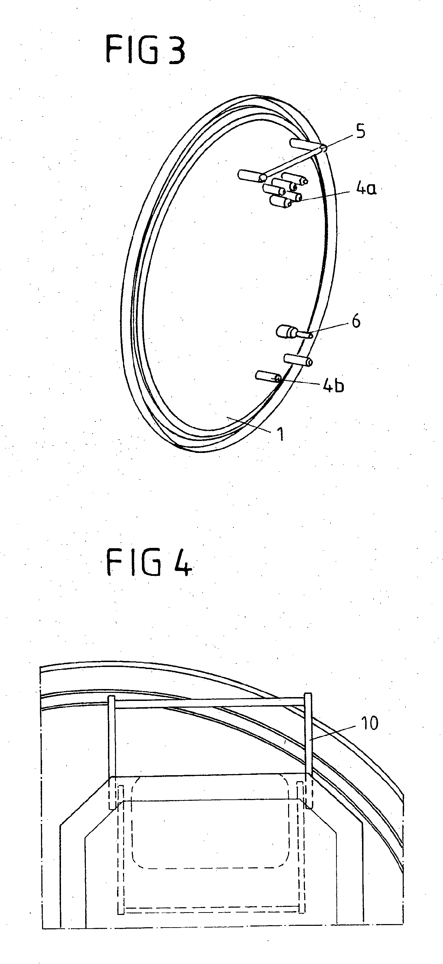

In Figur 3 ist eine bevorzugte Ausführungsform der erfindungsgemäßen Anordnung detailliert in einer Türronde einer Autoklaventür 1 dargestellt. In der hier gezeigten Ausführungsform besteht das Haltemittel 5 zum Halten des Behälters aus einer Haltevorrichtung aus zwei miteinander über einen Steg verbundenen Bolzen, die einstückig in die Türronde bzw. in das Material der Autoklaventür 1 eingebracht sind. Eine derartig gestaltete Haltevorrichtung erlaubt ein Einhaken des Instrumentenbehälters . 2 mittels an dem Instrumentenbehälter 2 vorgesehenen Haken.

Als besonders vorteilhaft hat sich erwiesen, wenn das Haltemittel 5 in Form eines Haltekörpers ausgestaltet ist, welcher eine Rotationsbewegung des Instrumentenbehälters 2 beim Anbringen desselbigen an der Autoklaventür 1 ermöglicht. Dies gilt insbesondere, wenn das Haltemittel bzw. die Haltevorrichtung 5 am oberen Drittel des Instrumentenbehälters 2 vorgesehen ist. Bei einer derartigen bevorzugten Lösung ist das Haltemittel derart gestaltet, dass ein Eingreifen des oberen Randes der Kassette in das Haltemittel bzw. die Haltevorrichtung erfolgt und der Instrumentenbehälter 2 anschließend durch eine Rotationsbewegung in die Endposition geführt wird. Die Rotationsbewegung des Instrumentenbehälters 2 unterstützt aufgrund der Gewichtskraft des Instrumentenbehälters 2 das Ankuppeln der Anschlussstücke 4a und 4b zum Ein- und Abführen der Fluiden in den Instrumentenbehälter 2.

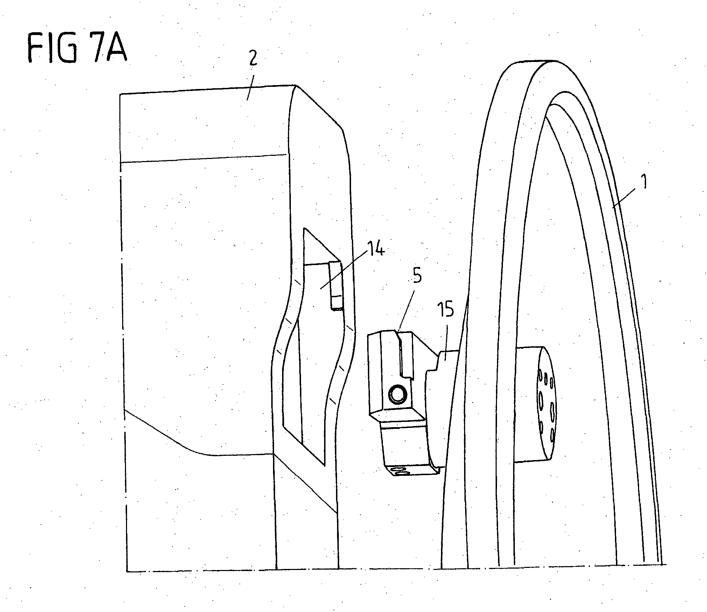

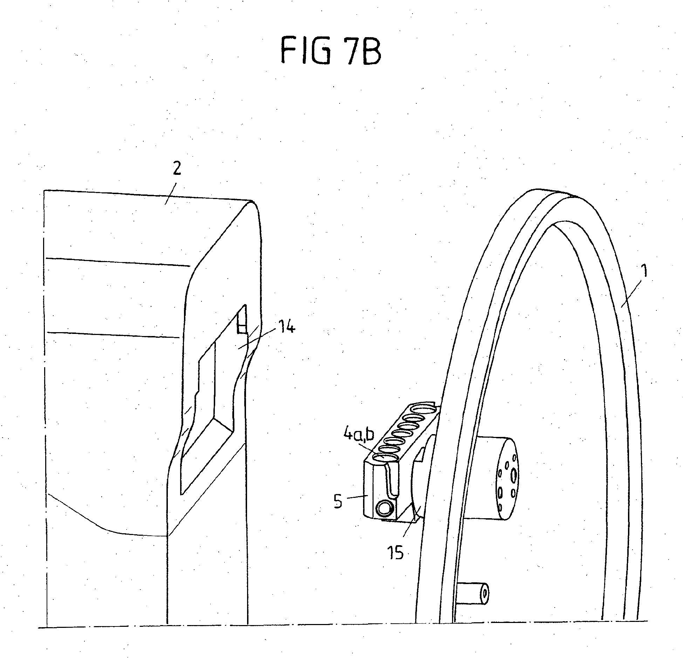

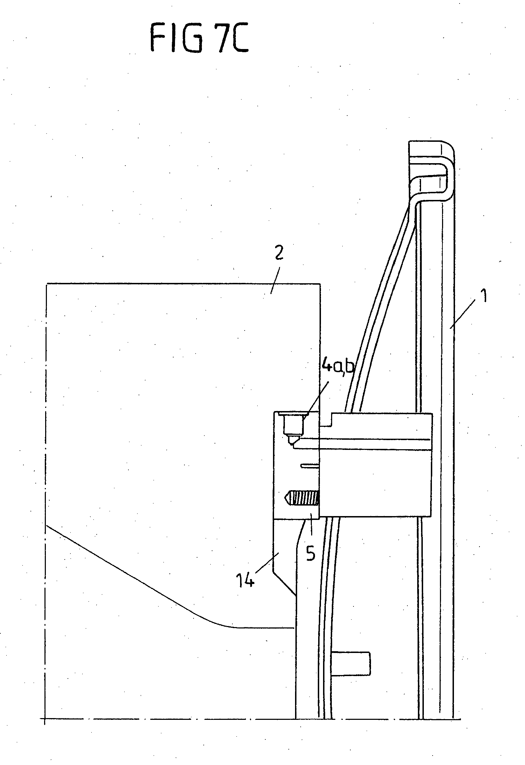

Eine weitere bevorzugte Ausführungsform eines geeigneten Haltemittels 5 ist in den Figuren 7a-c dargestellt. Hier besteht das Haltemittel 5 aus einem Kupplungsstück in welches die Anschlusstücke 4a, b z.B. im Falle der Fig 7b bis zu sieben Anschlussstücke integriert sind. Das Kupplungsstück kann aus einem Stück bestehen, bevorzugterweise aus Edelstahl oder geeigneten Kunststoffmaterial, welches mittels geeigneter Befestigungsmittel wie z.B. Schraube oder Bolzen mit dem beweglichen Teil 1, bevorzugt unter Verwendung eines zwischen Kupplungsstück und beweglichen Teil angeordneten Verbindungsteil 15 verbunden ist. Das Verbindungsteil 15 ist in die Autoklaventür 1 als beweglichem Teil integriert und umschließt die zu dem Anschlusstück 4a durch die Autoklaventür 1 führende Fluidleitung.

Die geometrische Form des Kupplungsstücks ist derart, dass dieses in ein am Instrumentenbehälter 2 vorgesehenes Haltemittel 14 z.B. in Form einer Aussparung eingreifen kann. Das zum Kupplungsstück korrespondierende Haltemittel 14 am Instrumentenbehälter 2 ist so gestaltet, dass der Instrumentenbehälter 2 zunächst in einer geraden Bewegung hin zum Kupplungsstück am beweglichen Teil 1 des Autoklaven bewegt wird (siehe Fig. 7a), wobei das Kupplungsstück in das korrespondierende Haltemittel 14 in Form einer sich- vom Instrumentenbehälter ausgesehen - nach oben hin verjüngenden Aussparung, d.h. entlang der vertikalen y-Achse des Instrumentenbehälters 2 schmaler werdenden Aussparung - eingeschoben wird, und der Instrumentenbehälter 2 anschließend senkrecht zur Einschubrichtung des Kupplungsstückes nach oben hin zur Verjüngung in der Aussparung 14 am Instrumentenbehälter 2 bewegt wird, wodurch sich der gewünschte Eingriff zwischen Haltemittel am Instrumentenbehälter und Kupplungsstück am beweglichen Teil ergibt (siehe Fig. 7b)

Gemäß der Ausführungsform der Figur 3 sind die Anschlussstücke 4a,b zum Ein- und Abführen der Fluiden in den Instrumentenbehälter 2 ebenfalls, wie auch das Haltemittel 5, einstückig mit der Türronde ausgebildet. Es ist generell aber auch möglich, dass sowohl Haltemittel 5 als auch Anschlussstücke 4a,b auf einer separaten Trägerplatte anordnbar sind. Im Falle einer einstückigen Ausbildung sind Haltemittel 5 und Anschlussstücke 4a,b bevorzugterweise aus demselben Material hergestellt, wie die Türronde der Autoklaventür 1. In der Ausführungsform der Figur 3 ist eine erste Variante 6 eines Mittels zum Verriegeln des Instrumentenbehälters 2 an der Autoklaventür 1 vorgesehen, das hier aus einem Drahtbügel besteht, der beim Anbringen des Instrumentenbehälters 2 in eine dafür am Instrumentenbehälter 2 vorgesehene Nut einschnappt und durch einen Hebel (nicht gezeigt) zum Entfernen des Instrumentenbehälters 2 gelöst wird. Das Mittel 6 kann auch zum Verbinden mit einem Sensor, der die Gegenwart des Instrumentenbehälters 2 detektiert, vorgesehen sein.

In Figur 4 ist eine besonders bevorzugte Ausführungsform der erfindungsgemäßen Anordnung dargestellt. Hier ist detailliert ein Mittel 10 zum Verriegeln des Instrumentenbehälters 2 in der Endposition an der Innenseite der Autoklaventür 1 dargestellt. Im vorliegenden Fall dient das Verriegelungsmittel 10 nicht nur einem Verriegeln des Instrumentenbehälters 2 in der gewünschten Endposition, sondern ermöglicht gleichzeitig die Führung und das Erkennen der korrekten Position des Instrumentenbehälters 2, es dient somit ebenfalls als Mittel zum Erkennen des korrekten Anbringens. Eine derartige Kombination von Erkennungsmittel und Verriegelungsmittel ist insbesondere dann von Vorteil, wenn diese Mittel der Prozesssteuerung gleichermaßen signalisieren, dass ein Aufbereitungsvorgang für die sich in dem Instrumentenbehälter 2 befindlichen Instrumente gestartet werden kann. Auf diese Weise wird sichergestellt, dass vom bedienenden Personal nicht voreiligerweise und fälschlicherweise ein Aufbereitungsprozess gestartet wird, ohne dass sich der Instrumentenbehälter 2 in der korrekten Endposition befindet, da nur in dieser korrekten Endposition eine sichere Zufuhr und Abfuhr der Fluide gewährleistet ist. Es ist dabei insbesondere von Vorteil, wenn die mechanische Verriegelung des Instrumentenbehälters 2 mit dem Auslösen eines elektrischen, kapazitiven, induktiven oder mechanischen Kontaktes einhergeht, der von der Prozesssteuerung entsprechend erkannt und verarbeitet wird, so dass der Sterilisations- und Reinigungsvorgang der sich im Instrumentenbehälter 2 befindlichen Instrumente gestartet werden kann.

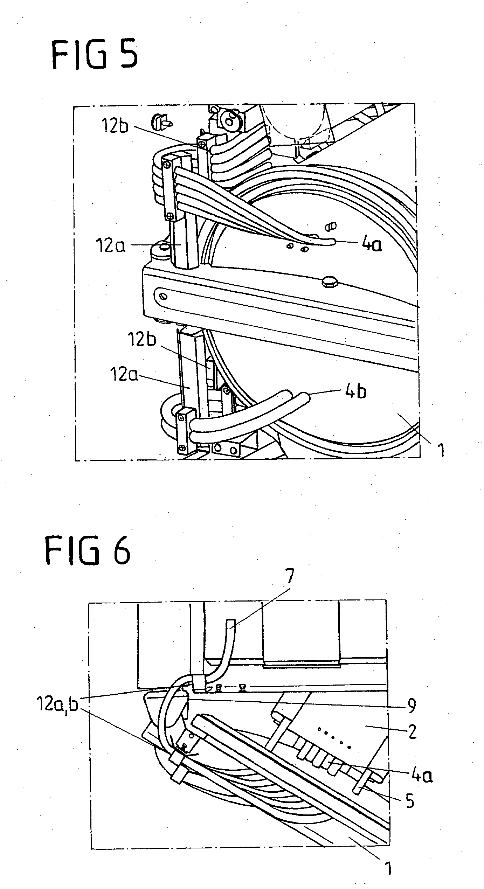

Figur 5 zeigt eine Ausführungsform der erfindungsgemäßen Anordnung, wobei vorliegend Schläuche 7 als Leitung zum Ein- und Abführen von Fluiden in den Instrumentenbehälter 2 vorgesehen sind. Wie Figur 5 entnehmbar, kann das Fluid, wie zum Beispiel Prozessmedien, über flexible Schläuche 7 als Fluidleitung aus dem Autoklavenkessel 3 in die Türverkleidung der Autoklaventür 1 transportiert werden. Die Schläuche 7 werden in der Ausführungsform der Figur 5 entlang des Autoklavenkessels 3 und entlang des Türscharniers 9 durch die Verkleidung der Autoklaventür 1 in den auf der Innenseite der Autoklaventür 1 angebrachten Instrumentenbehälter 2 (siehe auch Figur 6) geführt.

Da die Autoklaventür 1 ein bewegliches Teil darstellt, muss sichergestellt werden, dass die Schläuche 7 keinen starken Dehnungen oder Stauchungen unterliegen. Um eine Dehnung bzw. Stauchung zu verhindern, werden die Schläuche 7 an entsprechenden Halterungen 12a, 12b mittels geeigneter Befestigungsmittel befestigt. Dabei sind jeweils separate Halterungen für die fluidzuführenden Schläuche 7a und die fluidabführenden Schläuche 7b vorgesehen. Die jeweiligen Halterungen 12a sind unmittelbar am beweglichen Teil des Autoklaven, das heißt der Autoklaventür 1, angebracht und hier insbesondere in einem nahe zum Türscharnier angeordneten Bereich des Handgriffs 13 der Autoklaventür 1 angeordnet. Jeweilige zweite Halterungen 12b sind unmittelbar am vorderen Abschnitt des Autoklavenkessels 3, das heißt in einem Bereich des Autoklavenkessels 3 in unmittelbarer Nähe zum Türscharnier, angeordnet.

Das Führen der Schläuche entlang des Türscharniers und eine entsprechende Befestigung an dafür vorgesehenen Halterungen 12a, 12b ist insbesondere dann von Vorteil, wenn die Schläuche mindestens einseitig zugentlastet werden. Bei einer einseitigen Zugentlastung beschreiben die Schläuche 7 einen Halbkreis, wie der Draufsicht der Figur 6 entnehmbar. Besonders von Vorteil ist diese Anordnung und Befestigung der Schläuche 7 dann, wenn der Scheitelpunkt des von den Schläuchen 7 gebildeten Halbkreises genau in der Fluchtlinie des Scharniers liegt. In dieser Ausführungsform unter Verwendung von geeigneten Halterungen und Befestigungsmitteln unterliegen die Schläuche 7 nur noch einer zu vernachlässigenden Stauchung und Streckung bei den notwendigen Öffnungs- und Schließvorgängen der Autoklaventür 1.

Nach beendigter Reinigung und Sterilisation der in dem Instrumentenbehälter 2 angeordneten Instrumente, die typischerweise durch Einführen von geeigneten Reinigungsmitteln sowie Wasserdampf in den Instrumentenbehälter 2 durchgeführt wird, sind die Vorteile des Anbringens eines Instrumentenbehälters 2 an der Innenseite einer Autoklaventür 1 besonders eminent. Insbesondere ermöglicht das erfindungsgemäße Anbringen des Instrumentenbehälters 2 an der Autoklaventür 1 in Kombination mit einer automatischen Türöffnung ein deutliches Beschleunigen des Abkühlens der Instrumente in dem Instrumentenbehälter 2. Der Abkühlvorgang kann vorteilhafterweise noch durch die Verwendung eines zusätzlichen Kühlgebläses eines an der Autoklaventür 1 oder dem Autoklavenkessel 3 angeordneten zusätzlichen Kühlgebläses beschleunigt werden, welches einen Kühlmittelstrom in den Instrumentenbehälter 2 einführt.

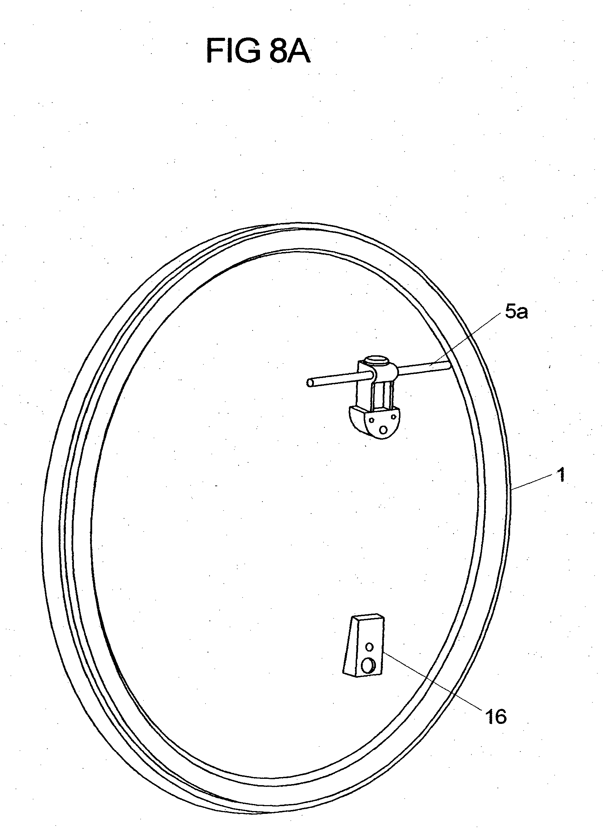

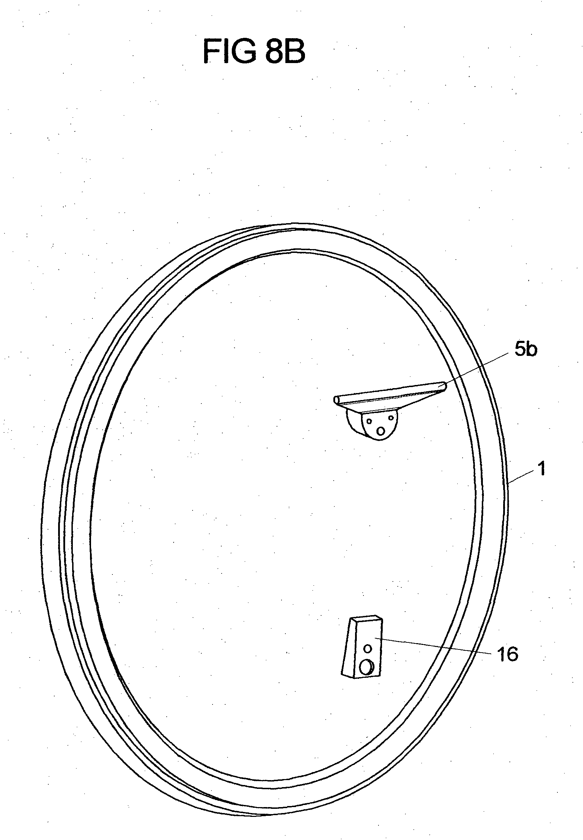

In den Figuren 8a und 8b sind weitere mögliche Ausführungsformen eines Haltemittels in Verbindung mit einem geeigneten Verriegelungsmittel 14 dargestellt. Gemäß der Ausführungsform des Haltemittels 5a der Figur 8a weist dieses eine Querstange zum Halten des Behälters 2 auf. Das Haltemittel 5a ist an der Türronde der Autoklaventür 1 befestigt. Zusätzlich zu dem Haltemittel 5a ist hier ein magnetisches Verriegelungsmittel 16 vorgesehen, welches eine magnetische Befestigung bzw. Verriegelung des Aufnahmebehälters 2 an der Türronde bewirkt. Das Haltemittel 5b der Figur 8a wiederum weist einen Haltegriff zum Halten des Behälters 2 auf. Auch in dieser Ausführungsform ist ein magnetisches Verriegelungsmittel 16 zur Befestigung bzw., Verriegelung des Aufnahmebehälters an der Türronde vorgesehen.

### Bezugszeichenliste

- 1: Autoklaventür
- 2: Instrumentenbehälter
- 3: Autoklavenkessel
- 4a,b: Anschlüsse zum Ein- und Abführen von Fluiden in und aus dem Instrumentenbehälter
- 5, 5a, 5b: Haltemittel
- 6: Erste Variante eines Verriegelungsmittels
- 7a, b: Fluidschläuche
- 8: Türverschluss
- 9: Türscharnier
- 10: Zweite Variante eines Verriegelungsmittels
- 11: Hebel
- 12a,b: Halterungselemente für Schläuche 7a,b
- 13: Handgriff an Autoklaventür
- 14: Haltemittel am Instrumentenbehälter
- 15: Verbindungsteil
- 16: Magnetisches Verriegelungsmittel

## Patentansprüche

1. Autoklav mit einem Autoklavenkessel (3) und einer Autoklaventür (1),
mindestens einen Behälter (2) für ärztliche und zahnärztliche Instrumente, der an einem in oder an der Autoklaventür (1) des Autoklaven (3) ausgebildetem Mittel zum Halten (5) des Behälters innerhalb des Autoklavenkessels (3) lösbar angebracht ist;
wobei der Behälter (2) als verschließbare Kammer ausgebildet ist, und
wobei Anschlüsse (4a) zum Einführen und Abführen von Fluiden in und aus dem Behälter (2) für ärztliche und zahnärztliche Instrumente in oder an der Autoklaventür (1) angeordnet sind;
**dadurch gekennzeichnet, dass**
das mindestens eine Haltemittel (5) in Form einer Querstange, von zwei miteinander über einen Steg verbundenen Bolzen, eines Bolzens, einer Schraube, einer Aussparung für Bajonetteingriff zwischen Haltemittel und Behälter, eines Kupplungsstückes, eines Haltekörpers oder einer Halteplatte ausgebildet ist.

2. Autoklav nach Anspruch 1, **dadurch gekennzeichnet, dass** der Autoklavenkessel (3) ein im Vergleich zu dem mindestens einen Behälter (2) für ärztliche und zahnärztliche Instrumente größeres Volumen aufweist.

3. Autoklav nach Anspruch 2, **dadurch gekennzeichnet, dass** das Verhältnis des Volumens des mindestens einen Behälters (2) für ärztliche und zahnärztliche Instrumente und Volumen des Autoklavenkessels (3) mindestens 1 : 1,7, bevorzugt 1:2,5, insbesondere bevorzugt 1:3,4 beträgt.

4. Autoklav nach einem der vorhergehende Ansprüche, **dadurch gekennzeichnet, dass** die Autoklaventür (1) den Zugriff in den Innenraum des Autoklavenkessels (3) ermöglicht und schwenkbar in Bezug auf den Autoklavenkessl (3) angeordnet ist.

5. Autoklav nach einem der vorhergehenden Ansprüche, **dadurch gekennzeichnet, dass** die Haltemittel (5) und die Anschlüsse (4a) zum Ein- und Abführen von Fluiden in aus dem Behälter (2) gemeinsam auf einer Trägerplatte oder auf separaten Trägerplatten angeordnet sind, wobei die Trägerplatte oder Trägerplatten jeweils an der Autoklaventür anbringbar und von dieser entfernbar sind.

6. Autoklav nach einem der Ansprüche 1 bis 4, **dadurch gekennzeichnet, dass** die Haltemittel (5) und die Anschlüsse (4a) zum Ein- und Abführen von Fluiden einstückig mit der Autoklaventür (1) ausgebildet sind.

7. Autoklav nach einem der vorhergehenden Ansprüche, **dadurch gekennzeichnet, dass** die in oder an der Autoklaventür vorgesehenen Anschlüsse (4a) zum Einführen und Abführen von Fluiden in und aus den Behälter (2) mit den am Behälter (2) vorgesehenen Anschlüssen (4b) zum Ein- und Abführen von Fluiden zum dichtenden Verbinden kompatibel sind.

8. Autoklav nach einem der vorhergehenden Ansprüche, **dadurch gekennzeichnet, dass** die in oder an der Autoklaventür (1) angeordneten Anschlüsse (4a) zum Einführen und Abführen von Fluiden in und aus dem Behälter (2) separate Stutzen oder ein kumuliertes Kupplungsstück umfassen.

9. Autoklav nach einem der vorhergehenden Ansprüche, **dadurch gekennzeichnet, dass** an der Autoklaventür (1) mindestens ein Erkennungsmittel (11) zum Erkennen der korrekten Position des Behälters (2) an der Autoklaventür (1) vorgesehen ist.

10. Autoklav nach einem der vorhergehenden Ansprüche, **gekennzeichnet durch** mindestens ein Verriegelungsmittel (10) zum Verriegeln des Behälters (2) in seiner zum Autoklavieren vorgesehenen Endposition an der Autoklaventür (1).

11. Autoklav nach einem der vorhergehenden Ansprüche, **dadurch gekennzeichnet, dass** das in den Behälter (2) ein- und ausgeführte Fluid durch mindestens eine Fluidleitung (7a,b) über die Anschlüsse (4a, 4b) in den Behälter (2) ein- und ausgeführt wird.

12. Autoklav nach Anspruch 11, **dadurch gekennzeichnet, dass** die mindestens eine Leitung (7a,b) an mindestens zwei Halterungen (12a, 12b) mittels geeigneter Befestigungsmittel befestigt ist.

13. Autoklav nach Anspruch 12, **dadurch gekennzeichnet, dass** mindestens eine erste Halterung (12a) an der Autoklaventür (1) und mindestens eine zweite Halterung (12b) am Autoklavenkessel (3) angeordnet sind.

14. Verfahren zur hygienischen Aufbereitung medizinischer und zahnmedizinischer Instrumente in einem Autoklaven nach einem der vorhergehenden Ansprüche **gekennzeichnet durch** die Schritte:
a) Einbringen von mindestens einem hygienisch aufzubereitenden medizinischen Instrument in einen Behälter (2) für medizinische und zahnmedizinische Instrumente;
b) Verbinden des Behälters (2) mit der mindestens einen in dem Autoklaven vorgesehenen Autoklaventür (1);
c) Durchführen der hygienischen Aufbereitung des sich in dem Behälter (2) befindlichen medizinischen Instruments;
d) nach Abkühlen Ablösen des Behälters (2) von der Autoklaventür (1); und
e) Entnahme des medizinischen Instruments aus dem Behälter (2).

## Claims

1. An autoclave with an autoclave kettle (3) and an autoclave door (1),
at least one container (2) for medical and dental instruments, which is releasably mounted on a means formed in or on the autoclave door (1) of the autoclave (3) for holding (5) the container within the autoclave kettle (3);
wherein the container (2) is formed as closable chamber, and
wherein ports (4a) for introducing and discharging fluids into and out of the container (2) for medical and dental instruments are arranged in or on the autoclave door (1);
**characterized in that**
the at least one holding means (5) is formed in the form of a cross bar, two bolts connected with each other via a web, a bolt, a screw, a cutout for bayonet engagement between holding means and container, a coupling piece, a holding body or a holding plate.

2. The autoclave according to claim 1, **characterized in that** the autoclave kettle (3) has a larger volume as compared to the at least one container (2) for medical and dental instruments.

3. The autoclave according to claim 2, **characterized in that** the ratio of the volume of the at least one container (2) for medical and dental instruments and the volume of the autoclave kettle (3) is at least 1:1.7, preferably 1:2.5, in particular preferably 1:3.4.

4. The autoclave according to any of the preceding claims, **characterized in that** the autoclave door (1) provides for access into the interior space of the autoclave kettle (3) and is pivotally arranged with respect to the autoclave kettle (3).

5. The autoclave according to any of the preceding claims, **characterized in that** the holding means (5) and the ports (4a) for introducing and discharging fluids into and out of the container (2) are jointly arranged on a carrier plate or on separate carrier plates, wherein the carrier plate or carrier plates each can be mounted on the autoclave door and can be removed from the same.

6. The autoclave according to any of claims 1 to 4, **characterized in that** the holding means (5) and the ports (4a) for introducing and discharging fluids are formed integrally with the autoclave door (1).

7. The autoclave according to any of the preceding claims, **characterized in that** the ports (4a) provided in or on the autoclave door for introducing and discharging fluids into and out of the container (2) are compatible with the ports (4b) provided on the container (2) for introducing and discharging fluids for the purpose of a sealing connection.

8. The autoclave according to any of the preceding claims, **characterized in that** the ports (4a) arranged in or on the autoclave door (1) for introducing and discharging fluids into and out of the container (2) comprise separate spouts or a cumulative coupling piece.

9. The autoclave according to any of the preceding claims, **characterized in that** on the autoclave door (1) at least one recognition means (11) is provided for recognizing the correct position of the container (2) on the autoclave door (1).

10. The autoclave according to any of the preceding claims, **characterized by** at least one locking means (10) for locking the container (2) in its end position on the autoclave door (1) provided for autoclaving purposes.

11. The autoclave according to any of the preceding claims, **characterized in that** the fluid introduced into and discharged from the container (2) is introduced into and discharged from the container (2) through at least one fluid line (7a, 7b) via the ports (4a, 4b).

12. The autoclave according to claim 11, **characterized in that** the at least one line (7a, 7b) is attached to at least two holders (12a, 12b) by means of suitable fastening means.

13. The autoclave according to claim 12, **characterized in that** at least one first holder (12a) is arranged on the autoclave door (1) and at least one second holder (12b) is arranged on the autoclave kettle (3).

14. A method for the hygienic treatment of medical and dental instruments in an autoclave according to any of the preceding claims, **characterized by** the steps:
a) introducing at least one medical instrument to be hygienically treated into a container (2) for medical and dental instruments;
b) connecting the container (2) with the at least one autoclave door (1) provided in the autoclave;
c) carrying out the hygienic treatment of the medical instrument present in the container (2);
d) after cooling releasing the container (2) from the autoclave door (1); and
e) removal of the medical instrument from the container (2).

## Revendications

1. Autoclave avec une chaudière d'autoclave (3) et une porte d'autoclave (1), au moins un conteneur (2) pour instruments médicaux et dentaires, qui est monté de manière amovible au niveau d'un moyen réalisé dans ou au niveau de la porte d'autoclave (1) de l'autoclave (3) pour retenir (5) le conteneur à l'intérieur de la chaudière d'autoclave (3) ;
dans lequel le conteneur (2) est réalisé en tant que chambre verrouillable, et
dans lequel des connecteurs (4a) pour l'introduction et l'évacuation de fluides dans et hors du conteneur (2) pour instruments médicaux et dentaires sont agencés dans ou au niveau de la porte d'autoclave (1) ;
**caractérisé en ce que** l'au moins un moyen de retenue (5) est réalisé sous la forme d'une barre transversale, de deux boulons reliés l'un à l'autre par le biais d'une traverse, d'un boulon, d'une vis, d'un évidement pour prise à baïonnette entre le moyen de retenue et le conteneur, d'une pièce de couplage, d'un corps de retenue ou d'une plaque de retenue.

2. Autoclave selon la revendication 1, **caractérisé en ce que** la chaudière d'autoclave (3) présente un volume plus grand par rapport à l'au moins un conteneur (2) pour instruments médicaux et dentaires.

3. Autoclave selon la revendication 2, **caractérisé en ce que** le rapport entre le volume de l'au moins un conteneur (2) pour instruments médicaux et dentaires et le volume de la chaudière d'autoclave (3) est au moins 1:1,7, de préférence 1:2,5, de manière particulièrement préférée 1:3,4.

4. Autoclave selon l'une quelconque des revendications précédentes, **caractérisé en ce que** la porte d'autoclave (1) permet l'accès à l'espace intérieur de la chaudière d'autoclave (3) et est agencée de manière pivotante par rapport à la chaudière d'autoclave (3).

5. Autoclave selon l'une quelconque des revendications précédentes, **caractérisé en ce que** les moyens de retenue (5) et les connecteurs (4a) pour l'introduction et l'évacuation de fluides dans hors du conteneur (2) sont agencés ensemble sur une plaque support ou sur des plaques support séparées, dans lequel la plaque support ou les plaques supports peuvent être montées respectivement sur la porte d'autoclave ou en être retirées.

6. Autoclave selon l'une quelconque des revendications 1 à 4, **caractérisé en ce que** les moyens de retenue (5) et les connecteurs (4a) pour l'introduction et l'évacuation de fluides sont réalisés d'un seul tenant avec la porte d'autoclave (1).

7. Autoclave selon l'une quelconque des revendications précédentes, **caractérisé en ce que** les connecteurs (4a) prévus dans ou au niveau de la porte d'autoclave pour l'introduction et l'évacuation de fluides dans et hors du conteneur (2) sont compatibles avec les connecteurs (4b) prévus au niveau du conteneur (2) pour l'introduction et l'évacuation de fluides pour le raccordement étanche.

8. Autoclave selon l'une quelconque des revendications précédentes, **caractérisé en ce que** les connecteurs (4a) agencés dans ou au niveau de la porte d'autoclave (1) pour l'introduction et l'évacuation de fluides dans et hors du conteneur (2) comprennent des raccords séparés ou un élément de couplage cumulé.

9. Autoclave selon l'une quelconque des revendications précédentes, **caractérisé en ce qu'**au moins un moyen de détection (11) pour la détection de la position correcte du conteneur (2) au niveau de la porte d'autoclave (1) est prévu au niveau de la porte d'autoclave (1).

10. Autoclave selon l'une quelconque des revendications précédentes, **caractérisé par** au moins un moyen de verrouillage (10) pour le verrouillage du conteneur (2) dans sa position finale prévue pour l'autoclavage au niveau de la porte d'autoclave (1).

11. Autoclave selon l'une quelconque des revendications précédentes, **caractérisé en ce que** le fluide introduit et évacué dans le conteneur (2) est introduit et évacué par au moins une conduite de fluide (7a, b) par le biais des connecteurs (4a, 4b) dans le conteneur (2).

12. Autoclave selon la revendication 11, **caractérisé en ce que** l'au moins une conduite (7a, b) est fixée à au moins deux fixations (12a, 12b) à l'aide de moyens de fixation adéquats.

13. Autoclave selon la revendication 12, **caractérisé en ce qu'**au moins une première fixation (12a) est agencée au niveau de la porte d'autoclave (1) et au moins une deuxième fixation (12b) est agencée au niveau de la chaudière d'autoclave (3).

14. Procédé de préparation hygiénique d'instruments médicaux et dentaires dans un autoclave selon l'une quelconque des revendications précédentes, **caractérisé par** les étapes de :
a) introduction d'au moins un instrument médical à préparer hygiéniquement dans un conteneur (2) pour instruments médicaux et dentaires ;
b) raccordement du conteneur (2) à l'au moins une porte d'autoclave (1) prévue dans l'autoclave ;
c) réalisation de la préparation hygiénique de l'instrument médical se trouvant dans le conteneur (2) ;
d) après refroidissement, désolidarisation du conteneur (2) de la porte d'autoclave (1) ; et
e) retrait de l'instrument médical du conteneur (2).
